(19) Europäisches Patentamt
European Patent Office
Office européen des brevets

(11) Veröffentlichungsnummer: **0 035 965**
**B1**

(12) # EUROPÄISCHE PATENTSCHRIFT

(45) Veröffentlichungstag der Patentschrift: **18.05.83**

(51) Int. Cl.³: **C 07 C 39/15**, C 07 C 37/00, C 07 C 37/11

(21) Anmeldenummer: **81810057.0**

(22) Anmeldetag: **23.02.81**

(54) **Verfahren zur Herstellung von 2,2'-Dihydroxybiphenyl-Verbindungen.**

(30) Priorität: **29.02.80 CH 1625/80**

(43) Veröffentlichungstag der Anmeldung: **16.09.81 Patentblatt 81/37**

(45) Bekanntmachung des Hinweises auf die Patenterteilung: **18.05.83 Patentblatt 83/20**

(84) Benannte Vertragsstaaten: **DE FR GB IT**

(56) Entgegenhaltungen:
**FR-A-2 332 252**

**Chemical Abstracts Band 37, Nr. 7, 10. April 1943 Columbus, Ohio, USA W.W. WESTERFELD et al. "The oxidation of p-cresol by peroxidase" Spalte 2025-4**

(73) Patentinhaber: **CIBA-GEIGY AG, Patentabteilung Postfach, CH-4002 Basel (CH)**

(72) Erfinder: **Rasberger, Michael, Dr., Waltersgrabenweg 6, CH-4125 Riehen (CH)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 2,2'-Dihydroxy-biphenyl-Verbindungen

Die vorliegende Erfindung betrifft ein neues Verfahren zum Herstellen von 2,2'-Dihydroxy-biphenyl-Verbindungen.

Es ist bekannt, 2,2'-Dihydroxy-biphenyle durch oxidative Kupplung aus den entsprechenden Phenolen herzustellen. So ist im US 2,885,444 die oxidative Kupplung von Phenolen mittels Sauerstoff beschrieben. Das Produkt wird nur dann in guten Ausbeuten und innerhalb kurzer Reaktionszeit gewonnen, wenn ein technisches Phenol in Gegenwart hoher Laugenkonzentrationen zum Einsatz kommt. Die Reaktion ist schwer kontrollierbar und führt daher zu schlecht reproduzierbaren Resultaten. Ferner ist das Produkt erst nach Zusatz von Säuren isolierbar. Der Einsatz von Sauerstoff in Gegenwart von organischen Lösungsmitteln ist ausserdem wegen der möglichen Bildung von explosiven Gemischen gefährlich. Es wurde daher der Sauerstoff durch Luft ersetzt. Eine technisch interessante Verfahrensführung ist dann allerdings nur in Gegenwart von Katalysatoren, wie Kupferpulver (US 2,785,188), Kupfersalzen (US 3,247,262), oder Kupfer-, Kobalt-, Mangan- oder Chrom-Komplexen von Diketonen oder Ketoester (US 4,097,461) möglich.

Die Wirkung von Wasserstoffperoxid auf oxidative Kupplungsreaktionen von Phenolen wurde ebenfalls untersucht. Westerfeld et al, Journal of Biological Chemistry 145, 463 (1942) hat gefunden, dass die Oxidation von Phenolen mittels Wasserstoffperoxid durch Peroxidase katalysiert werden kann. Das Hauptprodukt der Kupplung von p-Kresol ist allerdings das 2-Oxo-4a-dimethyl-1,2,4a,9b-tetrahydro-dibenzofuran, währenddem das gewünschte 2,2'-Dihydroxy-5,5'-dimethyl-diphenyl in einer Ausbeute von weniger als 5% bezogen auf das p-Kresol entsteht.

Es wurde nun ein Verfahren gefunden, welches es erlaubt, Phenole mittels Wasserstoffperoxid oxidativ zu kuppeln.

Die vorliegende Erfindung betrifft daher ein Verfahren zum Herstellen von 2,2'-Dihydroxy-biphenyl-Verbindungen der Formel I

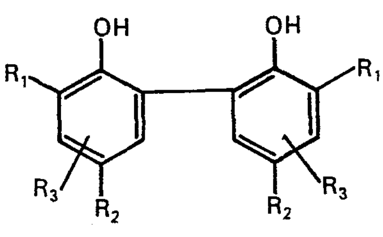

(I)

worin $R_1$ und $R_2$ unabhängig voneinander Wasserstoff, $C_1$-$C_{18}$ Alkyl, $C_2$-$C_6$ Alkenyl oder ggf. mit einem bis drei $C_1$-$C_4$ Alkylresten substituiertes $C_5$-$C_7$ Cycloalkyl, Phenyl oder $C_7$-$C_9$ Phenylalkyl bedeuten, oder $R_1$ mit $R_3$ zusammen einen an die 3,4- beziehungsweise 3',4'-Position gebundenen Butadi-1,3-enylen-1,4-Rest bedeutet, und $R_2$ ferner eine Gruppe $-(CH_2)_nCOOR_4$ ist, worin $R_4$ $C_1$-$C_{18}$ Alkyl undn 0, 1 oder 2 bedeuten, und $R_3$ Wasserstoff oder $C_1$-$C_{18}$ Alkyl ist, oder $R_2$ und $R_3$, falls $R_1$ Wasserstoff ist, einen über die 4,5- beziehungsweise 4',5'-Stellungen ankondensierten 1,1,3,3,-Tetra-methyl-propylenrest bedeuten, dadurch gekennzeichnet, dass man ein Phenol der Formel II

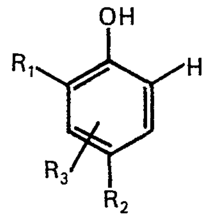

(II)

worin die Symbole $R_1$, $R_2$ und $R_3$, die oben angegebene Bedeutung haben, mittels Wasserstoffperoxid in Gegenwart einer starken anorganischen Base oxidativ kuppelt.

$R_1$, $R_2$, $R_3$ und $R_4$ sind als $C_1$-$C_{18}$ Alkyl geradkettiges oder verzweigtes Alkyl, wie Methyl, Äthyl, iso-Propyl, n-Butyl, n-Hexyl, 2-Äthylhexyl, n-Decyl, 1,1,3,3,5,5-Hexamethylhexyl, n-Tetradecyl oder n-Octadecyl. Insbesondere handelt es sich um Alkylreste mit 1-8 C-Atomen, wie Methyl, Äthyl, n-Propyl, iso-Propyl, sec. Butyl, t.Butyl, t.Amyl oder 1,1,3,3,-Tetramethylbutyl. $R_1$ ist in bevorzugten Phenolen der Formel II α-verzweigt. In besonders bevorzugten Phenolen der Formel II haben $R_1$ und $R_2$ dieselbe Bedeutung, z. B. t.Butyl oder 1,1,3,3-Tetramethylbutyl und $R_3$ ist Wasserstoff. Sind $R_1$ und $R_2$ ggf. mit 1 bis 3 Alkylgruppen substituiertes $C_5$-$C_7$ Cycloalkyl, so kann es sich um Cyclopentyl, o-Methylcyclopentyl, p-Butylcyclohexyl, 2,4,6-Trimethylcyclohexyl, Cyclohexyl, Cycloheptyl, 1-Methylcyclohexyl oder m-Äthylcyclohexyl handeln. Bevorzugt ist Cyclohexyl. $R_1$ und $R_2$ sind als $C_2$-$C_6$ Alkenyl zum Beispiel Vinyl, Allyl, 2-Butenyl oder 2-Hexenyl.

$R_1$ und $R_2$ sind als ggf. mit 1 bis 3 $C_1$-$C_4$ Alkylgruppen substituiertes Phenyl, beispielsweise 2,4-Dimethylphenyl, 4-Butylphenyl, 2,4,6-Triäthylphenyl und bevorzugt Phenyl.

$R_1$ und $R_2$ sind als ggf. mit 1 bis 3 $C_1$-$C_4$ Alkylgruppen substituiertes Phenylalkyl, zum Beispiel o-Methylbenzyl, p-Butylbenzyl, 2-Phenyläthyl, Benzyl oder insbesondere α,α-Dimethylbenzyl.

In bevorzugten Verbindungen bedeutet $R_3$ Wasserstoff und ist mindestens einer der Reste $R_1$ oder $R_2$ von Wasserstoff verschieden.

Bevorzugte Verbindungen, welche nach dem erfindungsgemässen Verfahren hergestellt werden können, entsprechen der Formel I, worin $R_1$ und $R_2$ Wasserstoff, $C_1$-$C_{18}$ Alkyl, Cyclohexyl, Phenyl oder α,α-Dimethylbenzyl sind und $R_3$ Wasserstoff ist oder zusammen mit $R_2$, falls $R_1$ Wasserstoff ist, ein über die 4,5- beziehungsweise 4',5'-Stellungen ankondensierter 1,1,3,3,-Tetramethylpropylenrest bedeutet.

Besondere Bedeutung haben erfindungsgemäss hergestellte Verbindungen der Formel I, worin eines von $R_1$ und $R_2$ $C_1$-$C_8$ Alkyl und das an-

dere Wasserstoff oder $C_1$–$C_8$ Alkyl bedeutet und $R_3$ Wasserstoff ist.

Beispiel von Phenolen der Formel II sind: Phenol, 2-Methylphenol, 2,4-Dimethylphenol, 2,4,-Di-t.-butylphenol, 2-Methyl-4-t.butylphenol, 2-t.Butyl-4-methylphenol, 2,4,-di-t.Amylphenol, 2-Isopropyl-4-t.butylphenol, 2-t.Butyl-3-äthyl-4-methylphenol, 2,5,-Dimethyl-4-t.butylphenol, 2,4-Di-(1′,1′,3′,3′-tetramethylbutyl)-phenol, 2,4,5-Trimethylphenol, 4-Nonylphenol, 4-Dodecylphenol, 2-t.Butyl-4-(1′,1′,3′,3′-tetramethyl)-phenol, 2-(1′,1′,3′,3′-Tetramethyl)-phenol, 2-Cyclohexylphenol, 2-Phenyl-4-t.butylphenol, 2-Benzylphenol, 2-(o-Methylcyclohexyl)-phenol oder 1,1,3,3,-Tetramethyl-5-hydroxy-2,3-dihydroindol.

Die erfindungsgemässe Reaktion verläuft bei Temperaturen zwischen 30 bis 100°C nahezu quantitativ. Die Reaktionszeiten sind sehr kurz und liegen bei Temperaturen zwischen 50 und 90°C unterhalb von 5 h. Die Reaktion kann bei einem Druck bis zu 30 atm durchgeführt werden. In der einfachsten und daher bevorzugten Verfahrensvariante wird allerdings kein Druck angewendet.

Das neue Verfahren ist nicht zuletzt deshalb technisch von Bedeutung, als dass relativ geringe Mengen an anorganischen Basen zum Einsatz kommen. Es hat sich gezeigt, dass 1,5 bis 4,0 Mol, bevorzugt 1,8 bis 3 Mol und insbesondere 2 Mol einer anorganischen Base bezogen auf 1 Mol des eingesetzten Phenols der Formel II ausreichen, um zu grosstechnisch sinnvollen Resultaten zu kommen. Grössere Basenmengen sind selbstverständlich auch möglich, doch ist es von grosser ökologischer Bedeutung, grosse Basenmengen zu vermeiden. Als starke anorganische Basen kommen insbesondere Alkali- und Erdalkali-hydroxide und -carbonate in Frage. Es handelt sich bevorzugt um Natrium- oder Kaliumhydroxid.

Das erfindungsgemässe Verfahren wird vorteilhaft in einem Lösungsmittel durchgeführt. Als Lösungsmittel haben sich besonders Wasser oder $C_1$-$C_4$-Alkohole, wie Methanol, Äthanol, Isopropanol oder Butanol, bzw. deren Gemische mit sich selbst oder mit Wasser, bewährt. Es eignen sich auch Kohlenwasserstoffe, wie Hexan, Benzol oder Toluol; chlorierte Kohlenwasserstoffe, wie Chloroform, Chlorbenzol oder Dichlorbenzol; und ferner Amide, wie Dimethylformamid oder Dimethylacetamid. Um zu den gewünschten hohen Raum-Zeit-Ausbeuten zu kommen, wird bevorzugt in möglichst konzentrierten Lösungen gearbeitet.

In der Regel wird Lauge und Phenol im Lösungsmittel vorgelegt, auf die Reaktionstemperatur erwärmt und dann das Wasserstoffperoxid zugetropft. Im allgemeinen werden wässrige handelsübliche $H_2O_2$-Lösungen verwendet, welche meist 30 bis 126 Vol-% Wasserstoffperoxid enthalten. Es werden in der Praxis stöchiometrische Mengen Wasserstoffperoxid eingesetzt, was bedeutet, dass das Volumen des Reaktionsgemisches nicht allzu stark zunimmt. Es kann auch ein Überschuss an $H_2O_2$ im Reaktionsmedium vorhanden sein.

Ein weiterer Vorteil dieser relativ konzentrierten Lösungen ist, dass der Kontakt mit dem Phenol rasch und intensiv ist, was zu den guten Raum-Zeit-Ausbeuten führt, wie sie mit gasförmigen Oxidationsmitteln nicht erreicht werden können.

Es ist bekannt, Reaktionen der vorliegenden Art oberflächenaktive Substanzen beizumengen, vor allem um feste Substanzen besser zu dispergieren, aber auch, um die Reinigung der Reaktionsgefässe zu vereinfachen. Oberflächenaktive Substanzen (dispersants, surfactants) sind organische Substanzen, welche in einem Molekül sowohl hydrophobe wie auch hydrophile Gruppen besitzen. Substanzen dieser Art sind beispielsweise in «Encyclopedia of Chemical Technology», Kirk-Othmer. 2nd Edition, Vol. 19 auf den Seiten 508–509 beschrieben. Alle dort erwähnten Verbindungen können im vorliegenden Verfahren eingesetzt werden. Die Einsatzkonzentration ist nicht kritisch und kann beispielsweise 0,0002 bis 0,4 Mol pro Mol Phenol betragen. Als besonders nützlich hat sich Natriumlaurylsulfat erwiesen.

Die Verbindungen der Formel I sind bekannte Zwischenprodukte, u.a. zur Herstellung von Antioxidantien oder Flammschutzmitteln für Kunststoffe.

Beispiel 1

40 g (1 Mol) Natriumhydroxid werden in 170 ml Wasser, das 1 g Natriumlaurylsulfat enthält, gelöst. Unter Rühren wird die Lösung auf 80°C erwärmt und 103 g (0,5 Mol) 2,4-Di-t.butylphenol zugefügt. Innerhalb von 2 Stunden werden 31 ml einer 30%igen Wasserstoffperoxid-Lösung zugetropft. Nach Abkühlen der Reaktionsmischung wird das Produkt abfiltriert und mit Wasser gewaschen. Das getrocknete Produkt 2,2′-Di-hydroxy-3,3′,5,5′-tetra-t.butyl-biphenyl (100,5 g: 98%) schmilzt bei 190–195°C.

Beispiel 1 bis 13

Auf analoge Weise erhält man:

2) 2,2′-Dihydroxy-3,3′,5,5′-tetra-t.amylbiphenyl (Fp 95°C)

3) 2,2′-Dihydroxy-3,3′,5,5′-tetra-(1,1,3,3-tetramethylbutyl)-biphenyl (Fp 145°C)

4) 2,2′-Dihydroxy-3,3′-di-t.butyl-5,5′-diisopropyl-biphenyl (Oel, $C_{ber}$ 81,6, $C_{gef}$ 81,2; $H_{ber}$ 10,01, $H_{gef}$ 10,00)

5) 2,2′-Dihydroxy-3,3′-di-t.butyl-5,5′-di-(1,1,3,3-tetramethylbutyl)-biphenyl (Oel, $C_{ber}$ 82, 69, $C_{gef}$ 82,2; $H_{ber}$ 11,18, $H_{gef}$ 11,00)

6) 1,1,3,3-Tetramethyl-5-hydroxy-6-(1′,1′,3′,3′-tetramethyl-5′-hydroxy-indan-6′-yl)-indan

7) 2,2′-Dihydroxy-3,3′-di-t.butyl-5,5′-octadecyloxy-propionyl-biphenyl

8) 2,2′-Dihydroxy-3,3′-di-t.butyl-5,5′-dionyl-biphenyl

9) 2,2′-Dihydroxy-3,3′-di-vinyl-5,5′-di-t.butyl-biphenyl

10) 2,2′-Dihydroxy-3,3′diphenyl-5,5′-dimethyl-biphenyl

11) 2,2'-Dihydroxy-3,3'-dicyclohexyl-5,5-di-t.butyl-biphenyl
12) 2,2'-Dihydroxy-3,3'-di-t.butyl-5,5'-dimethyl-biphenyl
13) 2-(1'-hydroxy-4'-t.butyl-2'-naphthyl)-4-t.butyl-1-naphthol.

**Patentansprüche**

1. Verfahren zum Herstellen von 2,2'-Dihydroxy-biphenyl-Verbindungen der Formel I

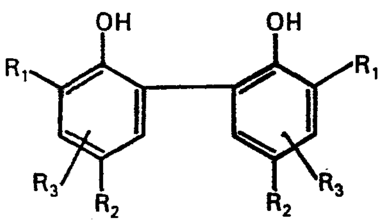

(I)

worin $R_1$ und $R_2$ unabhängig voneinander Wasserstoff, $C_1$-$C_{18}$ Alkyl, $C_2$-$C_6$ Alkenyl oder ggf. mit einem bis drei $C_1$-$C_4$ Alkylresten substituiertes $C_5$-$C_7$ Cycloalkyl, Phenyl oder $C_7$-$C_9$ Phenylalkyl bedeuten, oder $R_1$ mit $R_3$ zusammen einen an die 3,4- beziehungsweise 3',4'-Position gebundenen Butadi-1,3-enylen-1,4-Rest bedeutet, und $R_2$ ferner eine Gruppe $-(CH_2)_nCOOR_4$ ist, worin $R_4$ $C_1$-$C_{18}$ Alkyl und n 0, 1 oder 2 bedeutet, und $R_3$ Wasserstoff oder $C_1$-$C_{18}$ Alkyl ist, oder $R_2$ und $R_3$, falls $R_1$ Wasserstoff ist, einen über 4,5- beziehungsweise 4',5'-Stellungen ankondensierten 1,1,3,3-Tetra-methyl-propylenrest bedeuten, dadurch gekennzeichnet, dass man ein Phenol der Formel II

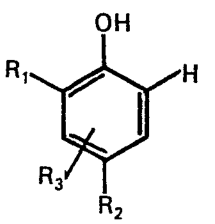

(II)

worin die Symbole $R_1$, $R_2$ und $R_3$ die oben angegebene Bedeutung haben, mittels Wasserstoffperoxid in Gegenwart einer starken anorganischen Base oxidativ kuppelt.

2. Verfahren nach Anspruch 1, wobei in der Formel I $R_1$ und $R_2$ Wasserstoff, $C_1$-$C_{18}$ Alkyl, Cyclohexyl, Phenyl oder $\alpha,\alpha$-Dimethylbenzyl sind, und $R_3$ Wasserstoff ist oder zusammen mit $R_2$, falls $R_1$ Wasserstoff ist, einen über die 4,5- beziehungsweise 4',5'-Stellungen ankondensierten 1,1,3,3-Tetramethyl-propylenrest bedeutet.

3. Verfahren nach Anspruch 1, wobei in der Formel I eines von $R_1$ und $R_2$ $C_1$-$C_{18}$ Alkyl und das andere Wasserstoff oder $C_1$-$C_8$ Alkyl bedeutet und $R_3$ Wasserstoff ist.

4. Verfahren nach Anspruch 1, dadurch gekennzeichnet, dass die Reaktion bei 30 bis 100°C abläuft.

5. Verfahren nach Anspruch 1, dadurch gekennzeichnet, dass die starke anorganische Base ein Alkali- oder Erdalkali-hydroxid oder -carbonat ist.

6. Verfahren nach Anspruch 1, dadurch gekennzeichnet, dass 1,5 bis 4,0 Mol einer starken anorganischen Base, bezogen auf das eingesetzte Phenol der Formel II, eingesetzt werden.

7. Verfahren nach Anspruch 1, dadurch gekennzeichnet, dass die Reaktion in einem Lösungsmittel durchgeführt wird.

8. Verfahren nach Anspruch 7, dadurch gekennzeichnet, dass die Reaktion in Wasser oder $C_1$-$C_4$ Alkohol, beziehungsweise deren Gemische mit sich selbst oder mit Wasser, durchgeführt wird.

9. Verfahren nach Anspruch 1, dadurch gekennzeichnet, dass das Wasserstoffperoxid als wässrige Lösung enthaltend 30–126 Vol% $H_2O_2$ eingesetzt wird.

10. Verfahren nach Anspruch 1, dadurch gekennzeichnet, dass die Reaktion in Gegenwart einer oberflächenaktiven Verbindung durchgeführt wird.

11. Verfahren nach Anspruch 10, dadurch gekennzeichnet, dass die Reaktion in Gegenwart von Natriumlaurylsulfat durchgeführt wird.

**Claims**

1. A process for the production of 2,2'-dihydroxy-biphenyl of the formula I

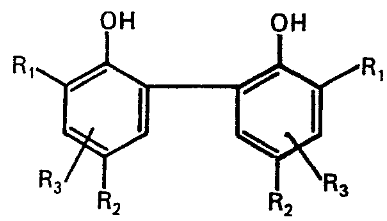

(I)

wherein each of $R_1$ and $R_2$ independently is hydrogen, $C_1$-$C_{18}$alkyl, $C_2$-$C_6$alkenyl, or$C_5$–$C_7$cycloalkyl, phenyl or $C_7$–$C_9$phenylalkyl, each of which is unsubstituted or substituted by one to three $C_1$–$C_4$alkyl radicals, or $R_1$ and $R_3$ together are a butadi-1,3-enyl-1,4-ene radical which is bonded to the benzene ring in the 3,4 and 3',4'-positions, and $R_2$ is also a $-(CH_2)_nCOOR_4$ group, wherein $R_4$ is$C_1$–$C_{18}$alkyl and n is 0, 1 or 2, and $R_3$ is hydrogen or $C_1$–$C_{18}$alkyl, or if $R_1$ is hydrogen, $R_2$ and $R_3$ are a 1,1,3,3-tetramethylpropylene radical which is bonded to the benzene ring in the 4,5- and 4',5'-positions, which process comprises oxidatively coupling a phenol of the formula II

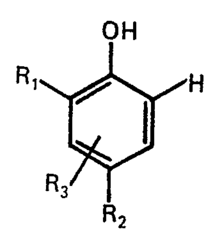

(II)

wherein $R_1$, $R_2$ and $R_3$ are as defined above, with hydrogen peroxide in the presence of a strong inorganic base.

2. A process according to claim 1, wherein $R_1$ and $R_2$ are hydrogen, $C_1$-$C_{18}$alkyl, cyclohexyl, phenyl, or $\alpha,\alpha$-dimethylbenzyl, and $R_3$ is hydrogen or, if $R_1$ is hydrogen, $R_2$ and $R_3$ together are a 1,1,3,3-tetramethylpropylene radical which is bonded to the benzene ring in the 4,5- and 4',5'-positions.

3. A process according to claim 1, wherein one of $R_1$ and $R_2$ in formula I is $C_1$-$C_8$alkyl and the other is hydrogen or $C_1$-$C_8$alkyl and $R_3$ is hydrogen.

4. A process according to claim 1, wherein the reaction is carried out in the temperature range from 30° to 100°C.

5. A process according to claim 1, wherein the strong inorganic base is a hydroxide or carbonate of an alkali metal or alkaline earth metal.

6. A process according to claim 1, wherein 1.5 to 4 moles of a strong inorganic base are employed, based on the phenol of the formula II.

7. A process according to claim 1, wherein the reaction is carried out in a solvent.

8. A process according to claim 7, wherein the reaction is carried out in water or a $C_1$-$C_4$alcohol, or in a mixture of such alcohols with each other or with water.

9. A process according to claim 1, wherein the hydrogen peroxide is employed in the form of an aqueous solution containing 30 to 126% by volume of $H_2O_2$.

10. A process according to claim 1, wherein the reaction is carried out in the presence of a surface-active compound.

11. A process according to claim 10, wherein the reaction is carried out in the presence of sodium lauryl sulfate.

**Revendications**

1. Procédé de préparation de dihydroxy-2,2' biphényles répondant à la formule I

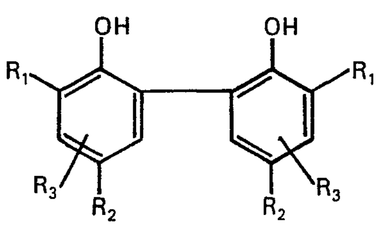

(I)

dans laquelle $R_1$ et $R_2$ représentent chacun, indépendamment l'un de l'autre, l'hydrogène, un radical alkyle en $C_1$–$C_{18}$, un radical alcényle en $C_2$–$C_6$ ou un radical cyclo-alkyle en $C_5$–$C_7$, phényle ou phénylalkyle en $C_7$–$C_9$ éventuellement porteur de 1 à 3 radicaux alkyles en $C_1$–$C_4$, ou $R_1$ et $R_3$ forment ensemble un radical butadiène-1,3 ylène-1,4 fixé en position 3,4 ou 3',4', et $R_2$ est en outre un radical $-(-CH_2)_nCOOR_4$ dans lequel $R_4$ représente un alkyle en $C_1$-$C_{18}$ et n un nombre égal à 0, à 1 ou à 2, et $R_3$ représente l'hydrogène ou un alkyle en $C_1$-$C_{18}$, ou $R_2$ et $R_3$, dans le cas où $R_1$ est l'hydrogène, représentent ensemble un radical tétraméthyl-1,1,3,3 propylène condensé en position 4,5 ou 4',5',

procédé caractérisé en ce qu'on unit par oxydation un phénol répondant à la formule II

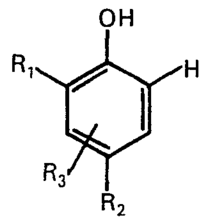

(II)

dans laquelle les symboles $R_1$, $R_2$ et $R_3$ ont les significations précédemment données, au moyen du peroxyde d'hydrogène, en présence d'une base minérale forte.

2. Procédé selon la revendication 1 caractérisé en ce que, dans la formule I, $R_1$ et $R_2$ représentent chacun l'hydrogène, un alkyle en $C_1$-$C_{18}$, un cyclohexyle, un phényle ou un $\alpha,\alpha$-diméthylbenzyle, et $R_3$ représente l'hydrogène ou forme avec $R_2$, dans le cas où $R_1$ représente l'hydrogène, un radical tétraméthyl-1,1,3,3 propylène condensé en position 4,5 ou 4',5'.

3. Procédé selon la revendication 1 caractérisé en ce que, dans la formule I, l'un des symboles $R_1$ et $R_2$ représente un alkyle en $C_1$–$C_8$, l'autre représente l'hydrogène ou un alkyle en $C_1$–$C_8$ et $R_3$ représente l'hydrogène.

4. Procédé selon la revendication 1 caractérisé en ce que la réaction est effectuée à une température comprise entre 30 et 100°C.

5. Procédé selon a revendication 1 caractérisé en ce que la base minérale forte est un hydroxyde ou un carbonate de métal alcalin ou de métal alcalino-terreux.

6. Procédé selon la revendication 1 caractérisé en ce qu'on utilise de 1,5 à 4,0 moles d'une base minérale forte, par rapport au phénol de formule II mis en jeu.

7. Procédé selon la revendication 1 caractérisé en ce qu'on effectue la réaction dans un solvant.

8. Procédé selon la revendication 7 caractérisé en ce qu'on effectue la réaction dans l'eau ou dans un alcool en $C_1$-$C_4$, ou encore dans des mélanges d'alcools de ce genre entre eux ou avec l'eau.

9. Procédé selon la revendication 1 caractérisé en ce que le peroxyde d'hydrogène est utilisé sous la forme d'une solution aqueuse contenant de 30 à 126% en volume d'$H_2O_2$.

10. Procédé selon la revendication 1 caractérisé en ce que la réaction est effectuée en présence d'un composé surfactif.

11. Procédé selon la revendication 10 caractérisé en ce que la réaction est effectuée en présence de sulfate de sodium et de lauryle.